(19) Europäisches Patentamt
European Patent Office
Office européen des brevets

(11) EP 0 528 856 B1

(12) EUROPEAN PATENT SPECIFICATION

(45) Date of publication and mention of the grant of the patent:
**02.04.1997 Bulletin 1997/14**

(21) Application number: **91908713.0**

(22) Date of filing: **22.04.1991**

(51) Int Cl.$^6$: **C07K 16/28**, C12P 21/04, A61K 39/395

(86) International application number:
**PCT/GB91/00633**

(87) International publication number:
**WO 91/16354 (31.10.1991 Gazette 1991/25)**

(54) **PROCESSES AND INTERMEDIATES FOR SYNTHETIC ANTIBODY DERIVATIVES**

VERFAHREN UND ZWISCHENPRODUKTE SYNTHETISCHER ANTIKÖRPERDERIVATE

PROCEDES ET INTERMEDIAIRES POUR PRODUIRE DES DERIVES D'ANTICORPS SYNTHETIQUES

(84) Designated Contracting States:
**AT BE CH DE DK ES FR GB GR IT LI LU NL SE**

(30) Priority: **23.04.1990 GB 9009106**

(43) Date of publication of application:
**03.03.1993 Bulletin 1993/09**

(73) Proprietor: **3i RESEARCH EXPLOITATION LIMITED**
**London SE1 8XP (GB)**

(72) Inventor: **STEVENSON, George, Telford**
**Southampton S01 7AP (GB)**

(74) Representative:
**Sheard, Andrew Gregory (GB) et al**
**Kilburn & Strode**
**30 John Street**
**GB-London WC1N 2DD (GB)**

(56) References cited:
**EP-A- 376 770**
**WO-A-90/04413**
**DE-A- 3 444 765**

- **Chemical Abstracts, vol. 110, No. 23, issued 1989, June 5 (Columbus, Ohio, USA); Stevenson, G.T. et al.: "A chimeric antibody with dual Fc regions (bisFabFc) prepared by manipulations at the IgG hinge", pages 552-553**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 528 856 B1

Printed by Jouve, 75001 PARIS (FR)

## Description

Co-pending, application PCT/GB89/01269 (published as WO 90/04413) relates to antibody derivatives having two or more Fc regions and to processes and intermediates for making such derivatives. This invention relates to alternative processes and intermediates having advantages in certain respects over those described in said earlier application.

Antibody derivatives with multiple Fc regions have a variety of possible therapeutic applications, especially when the Fc regions are linked to Fab regions or functionally similar moieties to form combinations having "chimeric" properties.

In order to construct such combinations in a controlled way it is advantageous to use, as intermediates, Fc regions having a controlled number of chemically functional groups which may be reacted with complementary functional groups on other moieties.

The present invention provides intermediates of this type, processes for making them and processes for using them in the manufacture of synthetic antibody derivatives.

Accordingly the present invention provides intermediates for use in the preparation of derivatives of Fc regions of antibodies, the said intermediates having the general formula:

Fc-S-S-X

where Fc is an Fc region in which external sulphydryl groups have been rendered substantially inert by incorporation into protein-protein disulphide bonds or by alkylation

and X is a protonated group causing the disulphide linkage is susceptible to reduction at a pH lower than that of other disulphide bonds.

By "external" we mean those sulphydryl groups released by reduction of interchain disulphide bonds. We distinguish these from sulphydryl groups arising from reduction of intrachain disulphide bonds: these intrachain bonds are buried internally in the protein, are difficult to reduce without substantially unfolding the protein and remain intact throughout all manipulations referred to in this application.

It will be understood by those skilled in the art that "inert" is not a completely absolute term. In this context we intend it to mean stable under process conditions to which the intermediate is to be subjected and in conditions of use, thus distinguishing from temporary masking groups and the like. Sulphydryl groups may, for example, be rendered inert by alkylation.

The group X is preferably pyridyl, permitting reduction at a low pH in the region of 4 where other disulphide bonds, for example inter-chain disulphide links and the like, are not reduced.

The term "Fc region" is intended to include not only, for example, typical immunoglobulin Fc structures comprising two peptide chains but also partial structures, active fragments and the like retaining Fc activity, e.g. recruitment effector ability or involvement in Ig transport and metabolism.

Fc regions may be derived from immunoglobulin of any immunoglobulin class or animal species but, if intended for human use, are preferably from human immunoglobulin, e.g. IgG or IgGl. Polyclonal or monoclonal sources may be used.

In general terms, intermediates of the present invention may be produced by the following process steps:

a) combining an Fc region with a "sacrificial" group;
b) subjecting the combination so formed to disulphide interchange and rendering potential reactive sites inert;
c) separating the sacrificial group from the Fc region to leave a reactive site on the Fc region; and
d) attaching a protonated group to the reactive site.

The sacrificial group is preferably selected from groups of sufficient molecular weight to enable separation of mono-substituted Fc regions from other reaction products by chromatographic fractionation or the like. One suitable group, for example, is an Fab' region from sheep immunoglobulin. This has the advantage of being a cheap and readily available source of immunoglobulin providing an Fab'$_2$ fragment with a single inter-gamma disulphide bond which can be cleaved to yield Fab'-SH and then Fab'-SS-Py with a single pyridyl disulphide. Another suitable group is human serum albumin.

Preferably the protonated group is pyridyl which may be attached to a reactive SH group by reacting with dipyridyl disulphide.

A more precise illustration of the method of the invention is the preparation of Fcγ -dithiopyridine (pyridyl-Fc, Fc-SS-Py), a stable derivative of human normal IgGl with a single pyridyl group protruding from a cysteine residue in the Fcγ hinge. When required for use Fc-SS-Py is quickly converted to its active form Fcγ -monomaleimide (Fc-mal), in

which the hinge cysteine bears a metastable maleimidyl group. This group enables the Fcγ to be linked to any protein with an accessible SH group, yielding chimeric derivatives joined by thioether bonds.

This preparation begins with papain digestion of human normal IgGI to yield Fcγ. An initial reduction of the Fcγ hinge releases four SH groups from the two parallel inter-γ SS bonds. One of these groups is protected by temporary incorporation into an SS link to a disposable protein (sacrificial group) (human albumin or sheep Fab'γ). The remaining three SH then undergo disulphide interchange with 4,4'-dipyridyl disulphide (Py-SS-Py), reconstituting an inter-γ SS from the paired SH groups and forming the mixed disulphide protein-SS-Py from the unpaired SH. This protein product is now exposed to dithiothreitol at pH 4.0 - where the inter-γ and Fcγ -albumin (or Fcγ -Fab'γ) SS bonds are not susceptible to reduction, but the bond in protein-SS-Py (highly electrophilic because of protonation of the pyridyl N) is. A solitary hinge SH results, and is then permanently blocked by alkylation with N-ethylmaleimide. The Fcγ -albumin chimera is purified chromatographically, cleaved by reduction of all interchain SS, and the albumin is discarded. The Fcγ with three hinge SH now undergoes a second round of disulphide interchange with Py-SS-Py to form the final Fc-SS-Py product, with a reconstituted hinge SS and a mixed disulphide (protein-SS-Py) formed from the unpaired SH.

The preparation may be simplified by omitting the first round of disulphide interchange with Py-SS-Py, proceeding instead to alkylate all three hinge SH after formation of the Fcγ -albumin chimera. The final Fc-SS-Py product then has an open hinge, which may be advantageous in certain situations (for example in not activating complement).

When required for use Fc-SS-Py is reduced with dithiothreitol at pH 4.0 to leave a solitary protein SH which, upon reaction with a molar surplus of a bismaleimide R-$(mal)_2$, forms Fcγ -S-succinimidyl-R-maleimide, i.e. Fc-mal.

The invention makes possible the production of a range of intermediate and final product structures and a variety of process steps and combinations. Such further possibilities will be readily understood by those skilled in the art and the present invention includes within its scope novel processes, intermediates and products derived from the invention whether as individual features or in combination with each other or further features to produce novel combinations.

Various aspects of the invention will now be exemplified in more detail with reference to the drawings in which:

Figure 1 illustrates the preparation of a precursor to the intermediate of the invention, of the form Fab-SS-Fc, in which a sacrificial Fab is combined with an Fc region having a sulphydryl group which has been rendered inert by alkylation (represented in the drawings by the _ symbol S°);

Figure 2 illustrates a subsequent stage in which the Fc region has been separated from the sacrificial Fab;

Figure 3 illustrates an intermediate (a) as provided by the invention and further process steps by which it may be converted into a form (c) for further reaction with another moiety; and

Figure 4 shows an Fab $Fc_2$ product of a type which may be constructed from the form illustrated in Figure 3(c).

Preparation of Fc-dithiopyridine (Fc-SS-Py)

Principles

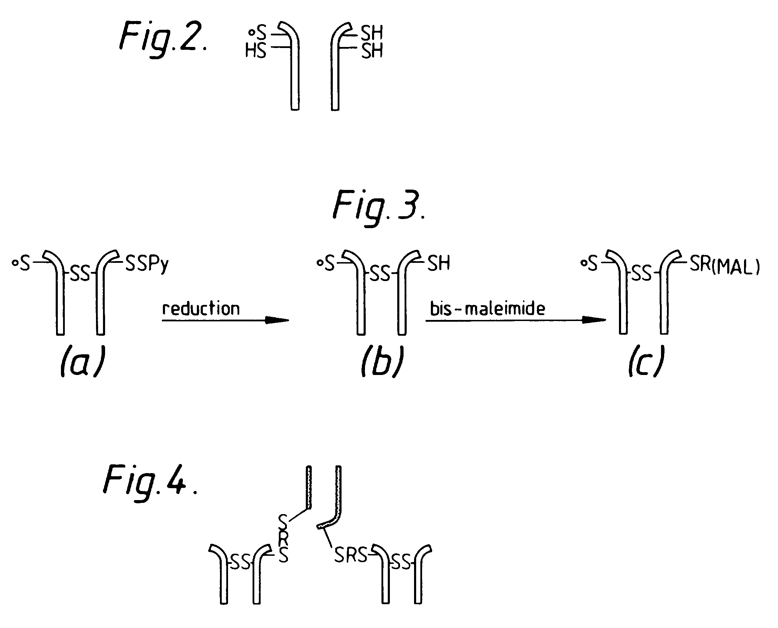

1. Human normal Fc gamma (see Figure 1, top right), from the predominant IgG subclass IgGI, is reduced so that its two hinge SS bonds yield four SH groups.
2. One of the Fc SH groups is used to form an SS bond with sheep Fab' gamma (see Figure 1, left), yielding the temporary species Fab-SS-Fc. Of the remaining Fc SH groups,two are now directed by SS-interchange to reform one of the original hinge SS bonds and one, directly opposite that utilised in the SS link to Fab, is blocked permanently by alkylation (see Figure 1, bottom right). Other possible reactions which may be used for blocking are discussed by Cecil and McPhee, Advances in Protein Chemistry 14:255, 1959.
3. Fab-SS-Fc is separated, by gel chromatography, from other products and surplus reagents in the (Fab + Fc) reaction mixture.
4. All interchain SS bonds in the Fab-SS-Fc are reduced to yield the structure of Figure 2, and the released Fab' gamma is discarded - having served its purpose of permitting the separation of Fc in which one of the four hinge SH has been blocked by alkylation.
5. A further round of SS-interchange again re-forms the available hinge SS, while the unpaired hinge SH is used to form a dithio(4-)pyridine group: (see Figure 3(a)).
6. The Fc-SS-Py is stored in this form. When required for use the S-SPy bond can be cleaved by gentle reduction at pH 4.0-4.5, and a free maleimide group (R) introduced by reaction with a large molar surplus of bismaleimide linker (e.g. o-phenylenedimaleimide), (see Figure 3(b) and 3(c). Reduction at pH 4.0-4.5 does not cleave the hinge SS. The susceptibility to reduction at acid pH shown by -SS-Py can be achieved using either 2-pyridyl or 4-pyridyl derivatives (the latter being the more susceptible), and is due to increased electrophilicity of the bond as the pyridyl N becomes protonated at the low pH (see Brockelhurst, Int. J. Biochem 10: 259, 1979).

Materials

Human Fc gamma is prepared from human normal IgG, itself obtained from plasma surplus to blood bank requirements, as described by Stevenson et al (Anti-Cancer Drug Design 3: 219, 1989).

Sheep IgG is prepared from serum by sequential precipitation with ammonium sulphate and DEAE-cellulose chromatography, and from it sheep F(ab' gamma)$_2$ is prepared by a standard peptic digestion (Nisonoff et al, Arch. Biochem. Biophys. 89: 230, 1960).

Dithiothreitol (DTT) and N-ethylmalemide (NEM), both from Sigma Chemical Co., are used without further purification.

4,4'-dipyridyl disulphide (Py-SS-Py) from Aldrich Chemical Co., is recrystallised from dimethylformamide/ water.

Sephadex, Sephacryl and Protein A-Sepharose chromatography media are from Pharmacia-LKB, Phospho-Ultrogel from IBF Biotechnics.

Details

The preparation is summarised in the flow-chart below. In this chart each "+" indicates a chemical reaction, each arrow the loading or unloading of a chromatographic column. The boxed products provide suitable subheadings for what follows.

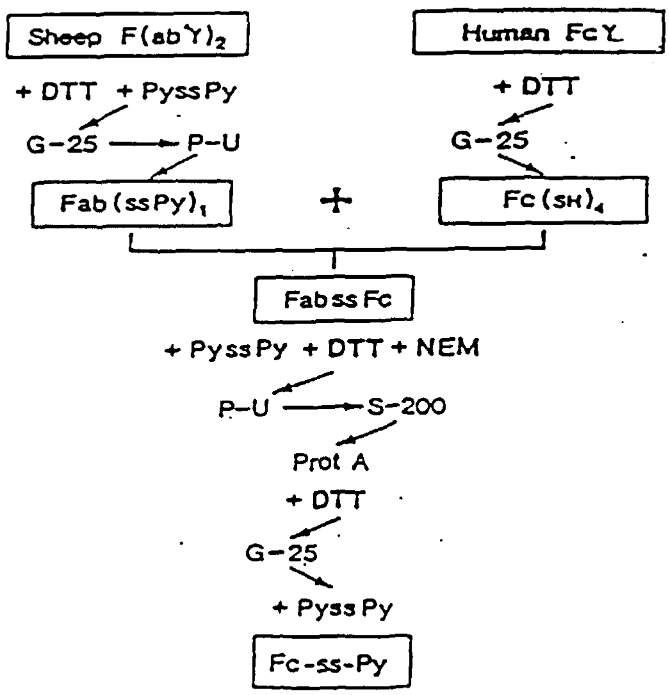


a. Fab (SSPy)$_1$

Sheep F(ab' gamma)2, 5mg.ml, is reduced with 1mM DTT at pH 8.0, 25°, for 30 minutes. It is then chilled and allowed to undergo SS-interchange at 5° for 15 minutes with Py-SS-Py added to 0.5mM surplus to the DTT. During this interchange the gamma-light SS bond reforms, while the SH derived from the solitary inter-gamma SS emerges as the mixed disulphide Fab-SS-Py. Passage through Sephadex G-25 removes all entities of low m.w. ( <5000) and transfers the protein to 0.03M acetate, pH 4.5 The protein is now led onto Phospho-Ultrogel A6R equilibrated with the same buffer, and binds to this medium in a compact zone (about 50 mg/cm$^3$). It is finally eluted from the gel at a concentration of about 30 mg/ml with 0.5M NaCl in the same acetate buffer.

b. Fc(SH)$_4$

Human Fc gamma at 20±2 mg/ml is reduced with 3mM DTT at pH 8.0, 25°, for 30 minutes. It is then transferred to 0.1M acetate, pH 4.0, with simultaneous removal of the DTT, by passage through Sephadex G-25.

c. Fab-SS-Fc

The Fab and Fc derivatives are mixed and incubated at 25° for 30 mins. During this period the principle reaction occurring is:

$Fab(SSPy)_1 + Fc(SH)_4 \rightarrow$ Fab-SS-Fc + 4-thiopyridone. There is also some formation of $Fab_2Fc$ and $Fab_3$ Fc.

At the end of the incubation the mixture is diluted with 2.8 volumes of cold water and Py-SS-Py is added to 0.17 mM. Disulphide-interchange reconstitutes those SS bonds in the Fc hinges where both cysteine SH remain free, while unpaired SH emerge as the mixed disulphide protein-SS-Py. The latter are reconverted to protein-SH by treatment with 0.4 mM DTT at pH 4.5. Then all further interchange is halted by alkylating the free SH upon addition of NEM to 2.9 mM.

The protein solution is now concentrated by absorption/desorption on Phospho-Ultrogel, as described for Fab $(SSPy)_1$, and the concentrated solution is led onto Sephacryl S200 equilibrated with 0.5M NaCl, 0.1 M acetate, pH 5.8. The Fab-SS-Fc separates from other proteins and is led onto, and bound to, Protein A-Sepharose equilibrated with the same buffer.

d. Fc-SS-Py

DTT, 1mM at pH 8.0, is led through the Protein-A-Sepharose column, thereby releasing the Fab' gamma for which the column has no affinity. After washing off the Fab' gamma the Fc, in the form $Fc(SH)_3$, is eluted with 0.1M glycine HCl, pH 3.0, and immediately transferred into 0.03 M acetate, pH 4.5, by passage through Sephadex G-25. Finally Py-SS-Py is added to 0.25 mM so that SS-interchange again restores the hinge SS and leaves the remaining non-alkylated cysteine as the mixed pyridyl disulphide (see Figure 3(a)). This intermediate has proved stable upon storage at 5°C for three months. The stored intermediate may be reduced and maleimidated as illustrated in Figure 3 (b) and (c) to give Fc-mal which may be linked to any agent with a free SH group, for example, $Fab(SH)_5$ to give $FabFc_2$ as illustrated in Figure 4.

In the above preparation of pyridyl Fc, sheep Fab'γ has been used to form a temporary species Fab-SS-Fc, thereby masking one SH group in the Fc hinge. At the same time the Fab-SS-Fc has a distinctive molecular weight (about 100,000), which allows it to be separated chromatographically from other reactants. These functions of sheep Fab'γ can be taken over by human serum albimin (HSA), so as to avoid additional safety precautions which might be required due to the introduction of a sheep protein. HSA has a single cysteine residue which can serve the masking function, and its molecular weight (64,500) will again endow the temporary species (HSA-SS-Fc) with a distinctive molecular weight for chromatographic separation.

Designate HSA with its single cysteine and hence its single SH group, as HSA $(SH)_1$. Then the overall reactions required are :

$$(1) \qquad HSA\ (SH)_1 + Py\text{-}SS\text{-}Py \rightarrow HSA(SS\text{-}pY)_1 + \text{thiopyridone}$$

$$(2) \qquad HSA\ (SS\text{-}Py)_1 + Fc(SH)_4 \rightarrow HSA\text{-}SS\text{-}Fc + \text{thiopyridone}$$

However, due to the fact that the SH group on HSA is relatively unreactive with other proteins, apparently not being readily accessible on the protein surface (see Cecil & McPhee, Advances in Protein Chemistry 14: 256, 1959), it has been found necessary to add an extension arm terminating in an SH to the cysteine residue before proceeding with reaction (1). Reactions (1) and (2) are then carried out as described above for sheep Fab'γ.

The preliminary steps required for HSA are as follows :

1. HSA (Sigma Chemical Company) is first reduced at pH 7.0 with dithiothreitol (2mM) to ensure that its cysteine residue is fully in the reduced (SH) form. It is then passed through Sephadex G-25 (Pharmacia), equlibrated with . 02M acetate buffer, pH 5.0 and run at 5°.

2. The protein now reacts with o-phenylenedimaleimide (1.2 mM, pH 5.0, dimethylformamide 16% v/v) at 5° for 60 minutes, to yield HSA $(mal)_1$ with an active maleimidyl group, and is again separated from reactants of low molecular weight by passage through Sephadex.

3. Finally the protein reacts again with dithiothreitol (1mM) to yield $HSA(SH)_1$, with its SH group located at the end of an arm about 15 Å long.

Advantages of the process of the invention compared with previous proposals are:

(1) The intermediate product is taken further along the road, giving a simpler and more obvious final preparative manoeuvre, and more readily suggesting further applications.
(2) The reaction Fc(mal) + $Fab(SH)_5$ proceeds more readily without sheep Fab persisting on the Fc to cause steric hindrance.

Possible uses of Fc-SS-Py

The intermediates of the invention may be stored and used, when required, to attach human Fc regions to any agent with a free SH group. The agent may be selected to home on abnormal tissue, such as cancer or virus-infected cells, and thereby coat the tissue with Fc in a manner which will attract the destructive power of effectors (complement, macrophages, NK cells) with affinities for the Fc.

Examples of agents suitable for coupling to human Fc:-

(1) The Fab' gamma region from a monoclonal antibody. Most antibodies readily yield this fragment upon peptic digestion. The chimeric antibody products so obtained could be used for:-

a. Killing cancer cells.
b. Suppressing host T lymphocytes in graft recipients.
c. Suppressing graft T lymphocytes in graft-vs-host disease.
d. killing virus-infected cells.
e. killing auto-immune lymphocytes (with antibody specific for the surface idiotypes, as for a class-specified antigen).

(2) Any other ligand for the surface of an undesirable cell, e.g. CD4, which will attach to the viral protein gP120 displayed on the surfaces of HIV-infected cells. Such a ligand must be able to display an SH group, and this might have to be introduced by genetic or chemical engineering.

**Claims**

**1.** Intermediates for use in the preparation of derivatives of Fc regions of antibodies, the said intermediates having the general formula:

Fc-S-S-X

where Fc is an Fc region in which external sulphydryl groups have been rendered substantially inert by incorporation into protein-protein disulphide bonds or by alkylation,
and - X is a protonated group, wherein the disulphide linkage is susceptible to reduction at a pH lower than that of other disulphide bonds.

**2.** Intermediates according to claim 1 characterised in that the disulphide linkage is susceptible to reduction at a pH in the region of 4.

**3.** Intermediates according to claim 2 characterised in that the group X is pyridyl.

**4.** Intermediates according to any one of claims 1 to 3 characterised in that the Fc region is from human immunoglobulin.

**5.** A process for the production of intermediates as claimed in any one of claims 1 to 4 having the following process steps :

a) combining an Fc region with a group selected from groups of sufficient molecular weight to enable separation of mono-substituted Fc regions from other reaction products;
b) subjecting the combination so formed to disulphide interchange and rendering potential reactive sites inert;
c) separating the group of (a) from the Fc region to leave a reactive site on the Fc region; and

d) attaching a protonated group to the reactive site.

6. A process according to claim 5 characterised in that the group in (a) is selected from groups of sufficient molecular weight to enable separation of mono-substituted Fc regions from other reaction products by fractionation.

7. A process according to claim 6 characterised in that the group is an Fab' region from sheep immunoglobulin.

8. A process according to claim 6 characterised in that the group is human serum albumin.

9. A process according to any one of claims 6 to 8 characterised in that the protonated group is pyridyl.

10. A process accroding to claim 9 characterised in that the pyridyl group is attached to the reactive site by reacting with dipyridyl disulphide.

11. The use of the intermediates as claimed in any one of claims 1 to 4 in a process for the preparation of derivatives of Fc regions of antibodies.

12. The use of the intermediates as claimed in any one of claims 1 to 4 in a process to attach a human Fc region to an agent with a free SH group.

13. Use according to claim 12 characterised in that the agent is selected to bind to abnormal tissue.

14. Use according to claim 12 or claim 13 characterised in that the agent is the Fab' gamma region from a monoclonal antibody.

15. Use according to claim 12 or claim 13 characterised in that the agent is a ligand for the surface of an undesirable cell.

16. Use according to any one of claims 12 to 15 in a process which includes a step wherein an intermediate is reduced.

17. The use of an intermediate as claimed in any one of claims 1 to 4 in the preparation of antibody derivatives.

18. The use as claimed in claim 17 wherein the antibody derivatives have 2 or more Fc regions.

**Patentansprüche**

1. Zwischenprodukte zur Verwendung bei der Herstellung von Derivaten von Antikörper-Fc-Regionen, wobei die Zwischenprodukte die allgemeine Formel:

$$Fc\text{-}S\text{-}S\text{-}X$$

aufweisen, wobei

- Fc eine Fc-Region ist, bei der äußere Sulfhydrylgruppen durch Einbau in Protein-Protein-Disulfidbrücken oder durch Alkylierung im wesentlichen inert gemacht wurden, und
- X eine protonierte Gruppe ist, bei der die Disulfidbindung bei niedrigerem pH-Wert für eine Reduktion zugänglich ist als andere Disulfidbrücken .

2. Zwischenprodukte nach Anspruch 1, dadurch gekennzeichnet, daß die Disulfidbindung bei einem pH-Wert von etwa 4 reduzierbar ist.

3. Zwischenprodukte nach Anspruch 2, dadurch gekennzeichnet, daß die Gruppe X Pyridyl ist.

4. Zwischenprodukte nach einem der Ansprüche 1 bis 3, dadurch gekennzeichnet, daß die Fc-Region von menschlichem Immunglobulin stammt.

5. Verfahren zur Herstellung von Zwischenprodukten nach einem der Ansprüche 1 bis 4 mit den folgenden Verfahrensschritten:

a) Verbinden einer Fc-Region mit einer Gruppe, die aus Gruppen von ausreichendem Molekulargewicht ausgewählt wird, um die Abtrennung von monosubstituierten Fc-Regionen von anderen Reaktionsprodukten zu ermöglichen;
b) Unterwerfen der so gebildeten Verbindung einem Disulfidaustausch und Inertisierung potentieller reaktiver Orte;
c) Abtrennen der Gruppe aus (a) von der Fc-Region, um einen reaktiven Ort auf der Fc-Region zu erhalten; und
d) Anlagern einer protonierten Gruppe an den reaktiven Ort.

**6.** Verfahren nach Anspruch 5, dadurch gekennzeichnet, daß die Gruppe in (a) aus Gruppen von ausreichendem Molekulargewicht ausgewählt wird, um die Abtrennung von monosubstituierten Fc-Regionen von anderen Reaktionsprodukten durch Fraktionierung zu ermöglichen.

**7.** Verfahren nach Anspruch 6, dadurch gekennzeichnet, daß die Gruppe eine Fab'-Region von einem Schafimmunglobulin ist.

**8.** Verfahren nach Anspruch 6, dadurch gekennzeichnet, daß die Gruppe menschliches Serumalbumin ist.

**9.** Verfahren nach einem der Ansprüche 6 bis 8, dadurch gekennzeichnet, daß die protonierte Gruppe Pyridyl ist.

**10.** Verfahren nach Anspruch 9, dadurch gekennzeichnet, daß die Pyridylgruppe an den reaktiven Ort durch Reaktion mit Dipyridyl-disulfid angelagert wird.

**11.** Verwendung des Zwischenprodukts nach einem der Ansprüche 1 bis 4 in einem Verfahren zur Herstellung von Derivaten von Antikörper-Fc-Regionen.

**12.** Verwendung der Zwischenprodukte nach einem der Ansprüche 1 bis 4 in einem Verfahren zur Bindung einer menschlichen Fc-Region an ein Agens mit einer freien SH-Gruppe.

**13.** Verwendung nach Anspruch 12, dadurch gekennzeichnet, daß das Agens ausgewählt wird, um an abnormes Gewebe zu binden.

**14.** Verwendung nach Anspruch 12 oder Anspruch 13, dadurch gekennzeichnet, daß das Agens die Fab'-Gammaregion eines monoklonalen Antikörpers ist.

**15.** Verwendung nach Anspruch 12 oder Anspruch 13, dadurch gekennzeichnet, daß das Agens ein Ligand für die Oberfläche einer unerwünschten Zelle ist.

**16.** Verwendung nach einem der Ansprüche 12 bis 15 in einem Verfahren, das einen Schritt beinhaltet, in dem ein Zwischenprodukt reduziert wird.

**17.** Verwendung eines Zwischenprodukts nach einem der Ansprüche 1 bis 4 bei der Herstellung von Antikörperderivaten.

**18.** Verwendung nach Anspruch 17, wobei die Antikörperderivate 2 oder mehr Fc-Regionen aufweisen.

**Revendications**

**1.** Intermédiaires à utiliser dans la préparation de dérivés de régions Fc d'anticorps, les intermédiaires ayant la formule générale :

Fc-S-S-X

où

Fc est une région Fc dans laquelle les radicaux sulfhydrile externes ont été rendus sensiblement inertes par incorporation dans des liaisons disulfure protéine-protéine ou par alcoylation, et
X est un radical protonné, dans lequel la liaison disulfure est susceptible d'être réduite à un pH inférieur à celui des autres liaisons disulfure.

**2.** Intermédiaires suivant la revendication 1, caractérisés en ce que la liaison disulfure est susceptible d'être réduite à un pH dans la région de 4.

**3.** Intermédiaires suivant la revendication 2, caractérisés en ce que le radical X est un pyridyle.

**4.** Intermédiaires suivant l'une quelconque des revendications 1 à 3, caractérisés en ce que la région Fc provient d'une immunoglobuline humaine.

**5.** Procédé de préparation des intermédiaires suivant l'une quelconque des revendications 1 à 4, comprenant les étapes suivantes :

(a) combinaison d'une région Fc avec un groupe choisi parmi les groupes de poids moléculaire suffisant pour permettre la séparation des régions Fc monosubstituées des autres produits de réaction;
(b) réalisation d'un échange de disulfure à la combinaison ainsi obtenue et conversion sous une forme inerte des sites potentiellement réactifs;
(c) séparation du groupe de (a) de la région Fc pour laisser un site réactif sur la région Fc, et
(d) fixation d'un radical protonné au site réactif.

**6.** Procédé suivant la revendication 5, caractérisé en ce que le groupe dans (a) est choisi parmi les groupes de poids moléculaire suffisant pour permettre la séparation des régions Fc monosubstituées des autres produits de réaction par fractionnement.

**7.** Procédé suivant la revendication 6, caractérisé en ce que le groupe est une région Fab' d'immunoglobuline de mouton.

**8.** Procédé suivant la revendication 6, caractérisé en ce que le groupe est la sérumalbumine humaine.

**9.** Procédé suivant l'une quelconque des revendications 6 à 8, caractérisé en ce que le radical protonné est un pyridyle.

**10.** Procédé suivant la revendication 9, caractérisé en ce que le radical pyridyle est fixé au site réactif par réaction avec du disulfure de dipyridyle.

**11.** Utilisation des intermédiaires suivant l'une quelconque des revendications 1 à 4, dans un procédé de préparation de dérivés de régions Fc d'anticorps.

**12.** Utilisation des intermédiaires suivant l'une quelconque des revendications 1 à 4, dans un procédé pour fixer une région Fc humaine à un agent avec un radical SH libre.

**13.** Utilisation suivant la revendication 12, caractérisée en ce que l'agent est choisi pour qu'il se fixe à des tissus anormaux.

**14.** Utilisation suivant la revendication 12 ou 13, caractérisée en ce que l'agent est la région gamma Fab' d'un anticorps monoclonal.

**15.** Utilisation suivant la revendication 12 ou 13, caractérisée en ce que l'agent est un ligand de surface d'une cellule indésirable.

**16.** Utilisation suivant l'une quelconque des revendications 12 à 15, dans un procédé qui comprend une étape dans laquelle on réduit un intermédiaire.

**17.** Utilisation d'un intermédiaire suivant l'une quelconque des revendications 1 à 4, dans la préparation de dérivés d'anticorps.

18. Utilisation suivant la revendication 17, dans laquelle les dérivés d'anticorps ont 2 régions Fc ou davantage.

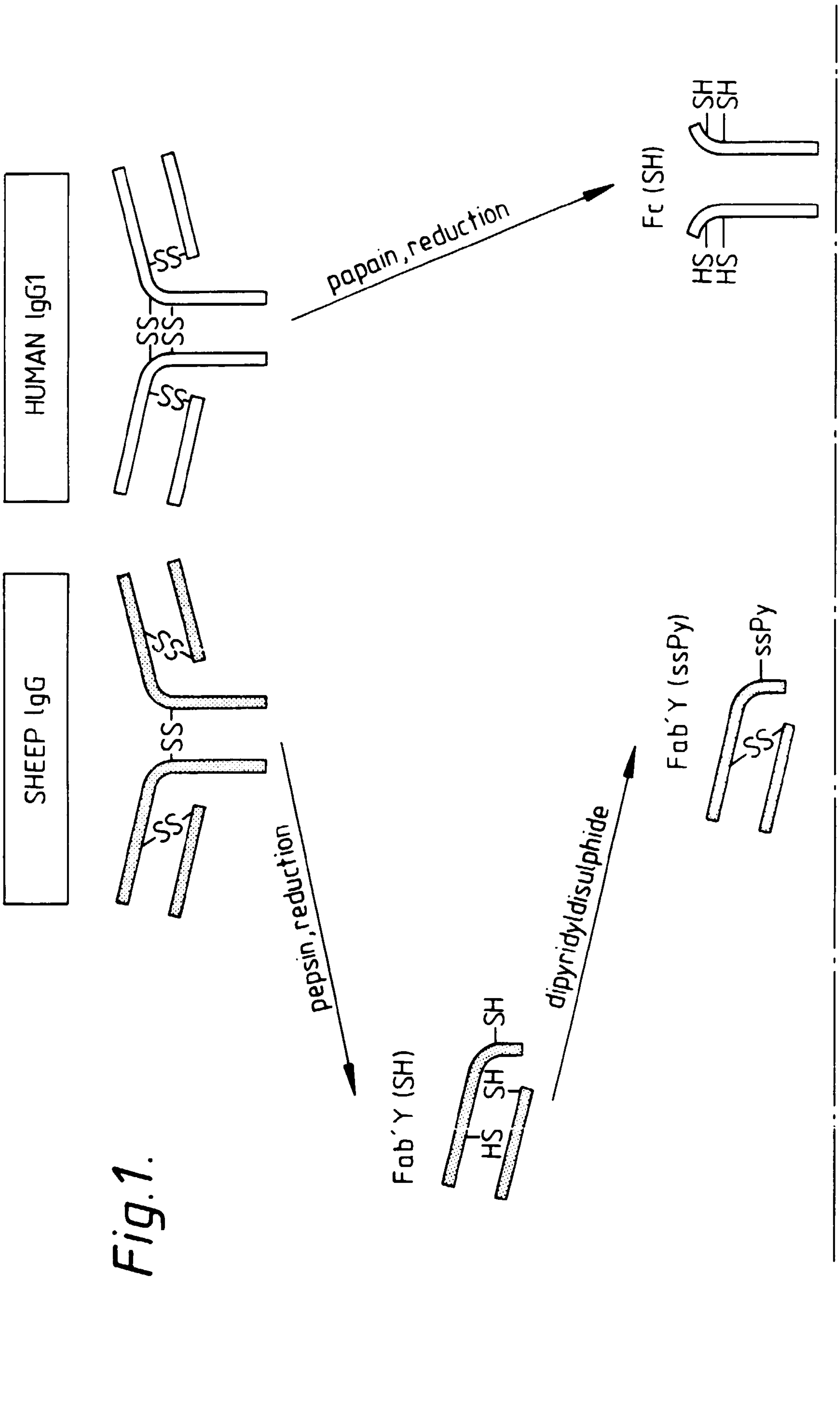
HUMAN IgG1
SHEEP IgG
SS
papain, reduction
pepsin, reduction
Fc (SH)
HS
SH
Fab'Y (SH)
dipyridyldisulphide
Fab'Y (ssPy)
ssPy

Fig.1.

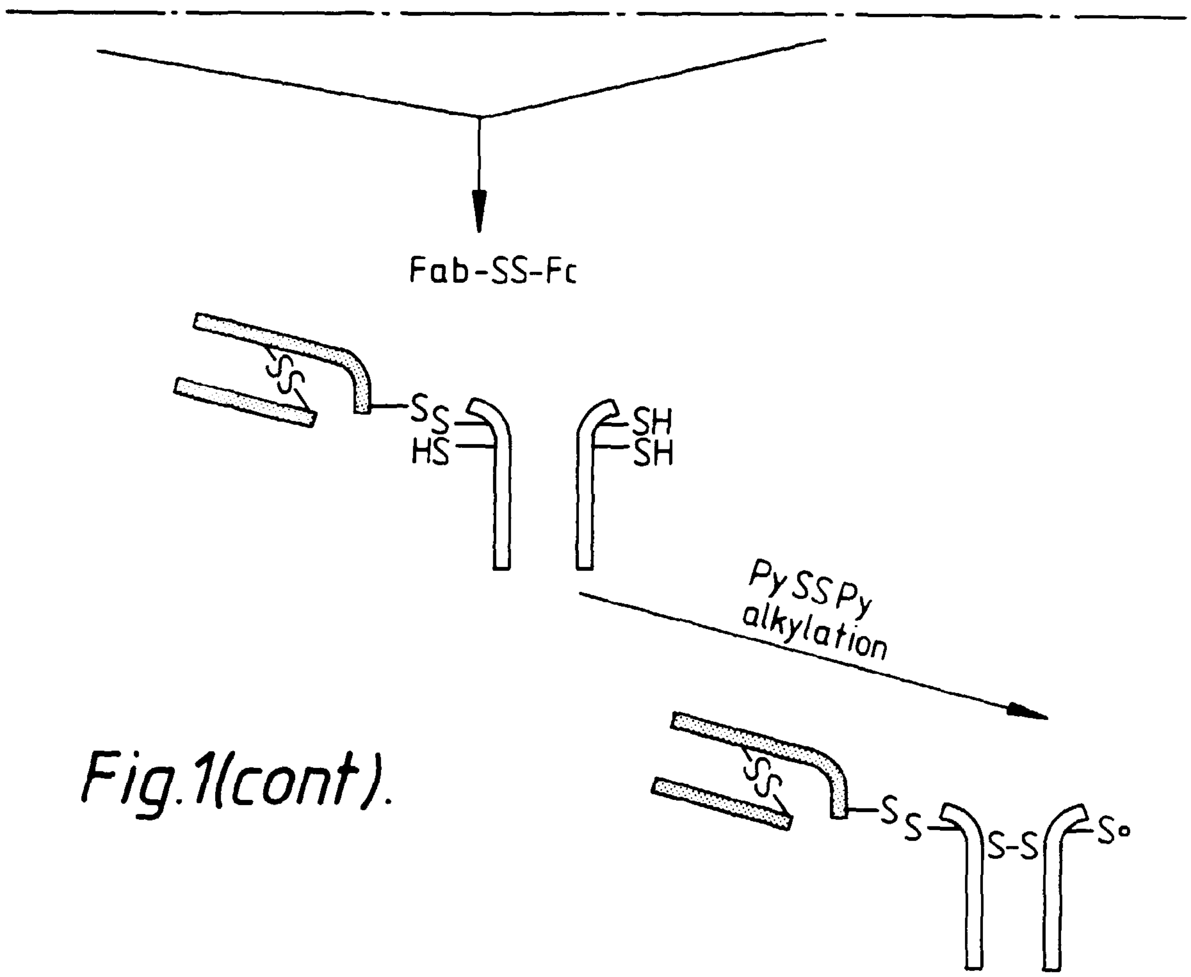


Fig.1(cont).

Fig.2.

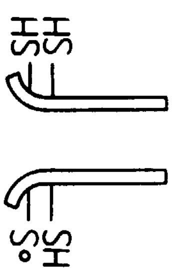


Fig.3.

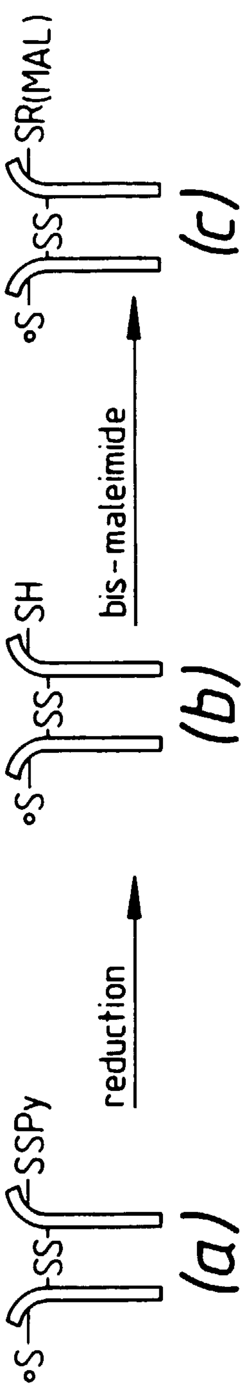


Fig.4.